(19) Europäisches Patentamt
European Patent Office
Office européen des brevets

(11) Veröffentlichungsnummer: **0 046 191**
**A1**

(12) # EUROPÄISCHE PATENTANMELDUNG

(21) Anmeldenummer: **81105448.5**

(22) Anmeldetag: **13.07.81**

(51) Int. Cl.³: **C 07 D 261/20**

(30) Priorität: **20.08.80 DE 3031324**

(43) Veröffentlichungstag der Anmeldung: **24.02.82**
**Patentblatt 82/8**

(84) Benannte Vertragsstaaten: **BE CH DE FR GB LI**

(71) Anmelder: **BASF Aktiengesellschaft,**
**Carl-Bosch-Strasse 38, D-6700 Ludwigshafen (DE)**

(72) Erfinder: **Rieber, Norbert, Dr., Liebfrauenstrasse 1c,**
**D-6800 Mannheim (DE)**
Erfinder: **Boehm, Heinrich, Dr., Keplerweg 2,**
**D-6701 Neuhofen (DE)**
Erfinder: **Platz, Rolf, Dr., Hansastrasse 5,**
**D-6800 Mannheim (DE)**
Erfinder: **Fuchs, Werner, Dr., Muenchbuschweg 30B,**
**D-6700 Ludwigshafen (DE)**

(54) **Isoxazolinverbindungen und Verfahren zu ihrer Herstellung.**

(57) Neue Isoxazolinverbindungen und ein Verfahren zu ihrer Herstellung durch Umsetzung von Isoxazolin-azoverbindungen bei einer Temperatur von 50 bis 200 °C.

Die nach dem Verfahren der Erfindung herstellbaren Endstoffe sind wertvolle Ausgangsstoffe für die Herstellung von Farbstoffen, Pflanzenschutzmitteln und Pharmazeutika.

EP 0 046 191 A1

## Isoxazolinverbindungen und Verfahren zu ihrer Herstellung

Die Erfindung betrifft neue Isoxazolinverbindungen und ein Verfahren zu ihrer Herstellung durch Umsetzung von Isoxazolin-azoverbindungen bei einer Temperatur von 50 bis 200°C.

Es ist aus Chem. Communications, Band 1969, Seite 72, bekannt, daß man die Bis-Azoverbindung der Formel

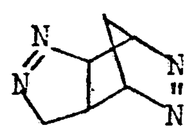

durch Erhitzen unter Stickstoff während 5 Stunden auf 120°C in Pyrazol und ein tricyclisches Pyrazolinderivat im Mengenverhältnis 25 : 75 zerlegen kann:

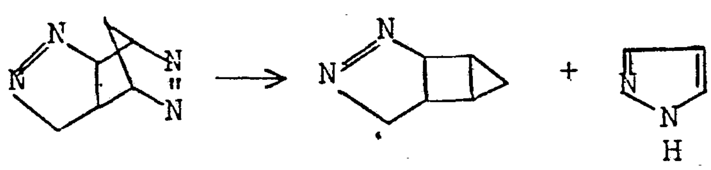

Es wurde nun gefunden, daß man Isoxazolinverbindungen der Formel

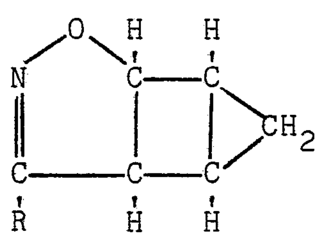 I,

worin R ein Wasserstoffatom, einen aliphatischen, cycloaliphatischen, araliphatischen oder aromatischen Rest bedeutet, vorteilhaft erhält, wenn man Isoxazolin-azoverbin-WB/BL

dungen der Formel

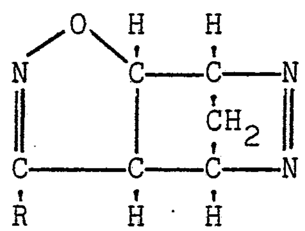

II,

worin R die vorgenannte Bedeutung besitzt, bei einer Temperatur von 50 bis 200°C umsetzt.

Weiterhin wurden die neuen Isoxazolinverbindungen der Formel

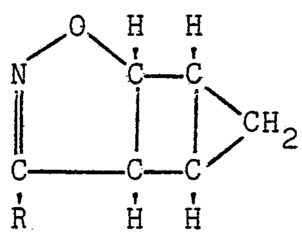

I,

worin R ein Wasserstoffatom, einen aliphatischen, cycloaliphatischen, araliphatischen oder aromatischen Rest bedeutet, gefunden.

Die Umsetzung kann für den Fall der Verwendung von 2,3,7-Triaza-6-oxa-tricyclo-$[5.2.1.0^{5.9}]$-deca-2,7-dien durch die folgenden Formeln wiedergegeben werden:

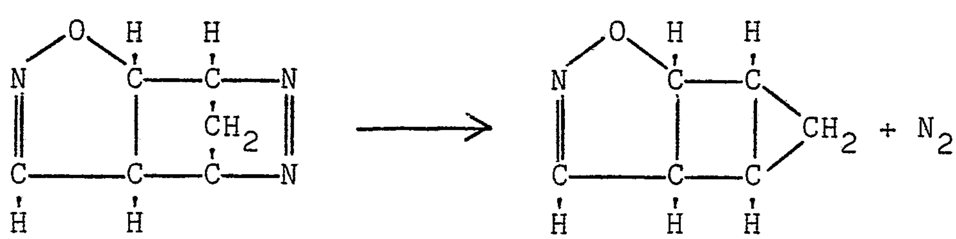

Das Verfahren nach der Erfindung liefert auf einfachem und

wirtschaftlichem Wege die neuen Isoxazolinverbindungen in guter Ausbeute und Reinheit. Alle diese vorteilhaften Ergebnisse sind mit Bezug auf den Stand der Technik überraschend.

Bevorzugte Ausgangsstoffe II und demzufolge bevorzugte Endstoffe I sind solche, in deren Formeln R ein Wasserstoffatom, einen Alkylrest mit 1 bis 18, insbesondere 1 bis 6 Kohlenstoffatomen, einen Cycloalkylrest mit 3 bis 8 Kohlenstoffatomen, einen Aralkylrest mit 7 bis 12 Kohlenstoffatomen, einen unsubstituierten oder durch Bromatome, Fluoratome, Chloratome, Hydroxygruppen, Trifluormethylgruppen, Nitrogruppen, Dialkylaminogruppen, Alkylgruppen, Alkoxygruppen und/oder durch Fluor substituierte Alkoxygruppen mit jeweils 1 bis 4 Kohlenstoffatomen pro Alkylgruppe substituierten Phenylrest bedeutet. Die vorgenannten Reste können noch durch unter den Reaktionsbedingungen inerte Gruppen und/oder Atome, z.B. Dialkylamino-, Monoalkylaminogruppen, Alkylgruppen, Trifluormethylgruppen, Alkylthiogruppen, Alkoxygruppen und durch Fluor substituierte Alkoxygruppen mit jeweils 1 bis 4 Kohlenstoffatomen je Alkylgruppe, Phenylreste substituierende Fluoratome, Bromatome, Chloratome, Oxogruppen, Carboxygruppen, Sulfonsäuregruppen, substituiert sein. Die Ausgangsstoffe II können leicht durch Umsetzung in einem ersten Schritt von Nitriloxiden der Formel

$$R^1CNO \quad III,$$

worin $R^1$ die vorgenannte allgemeine und bevorzugte Bedeutung besitzt, mit N,N'-Dicarbalkoxy-2,3-diaza-bicyclo-[2,2,1]-hept-2-enen der Formel

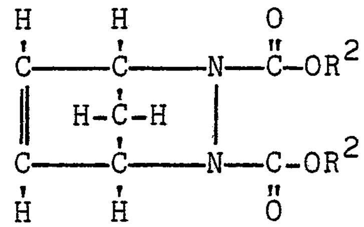 IV,

worin die einzelnen Reste $R^2$ gleich oder verschieden sein können und jeweils ein Wasserstoffatom oder einen aliphatischen Rest bedeuten, und dann in einem 2. Schritt die so erhaltenen N,N'-Dicarbalkoxy-isoxazolino-hydrazoverbindungen der Formel

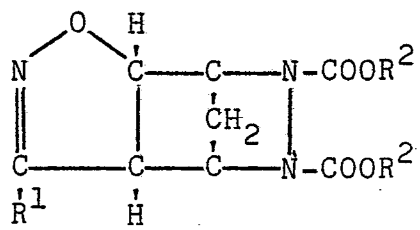 V,

worin $R^1$ und $R^2$ die vorgenannten Bedeutungen besitzen, nach üblicher Weise verseift, decarboxyliert und zu den Ausgangsstoffen II oxidiert.

Die Ausgangsstoffe III sind leicht durch die in Houben-Weyl, Methoden der Organischen Chemie, Band 10/3, Seiten 841 bis 853 beschriebenen Arbeitsweise, z.B. durch Dehydrierung von Aldoximen oder aus Hydroxamsäurederivaten oder Nitrolsäuren zugänglich. Die Ausgangsstoffe IV erhält man z.B. durch Umsetzung von Cyclopentadien mit Azodicarbonsäuredialkylestern nach den in Ann. 443, 249 - 262 (1925) beschriebenen Verfahren. Man erhält die Endstoffe I in Form ihrer Isomere.

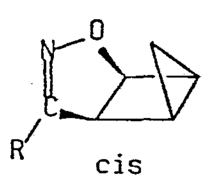
cis

und

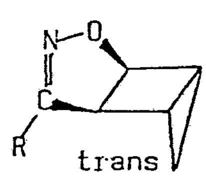
trans

So kommen beispielsweise die folgenden Ausgangsstoffe II infrage: 2,3,7-Triaza-6-oxa-tricyclo-$[5.2.1.0^{5.9}]$-deca-2,7-dien und seine in 8-Stellung durch die Methyl-, Äthyl- Propyl-, Isopropyl-, Butyl-, Isobutyl-, sek.-Butyl-, tert.-Butyl-, Heptyl-, Cyclohexyl-, Cyclopentyl-, Cyclopropyl-, Benzyl-, Phenyl-, 2-Chlorphenyl-, 3-Chlorphenyl-, 4-Chlorphenyl-, 2-Methylphenyl-, 3-Methylphenyl-, 4-Methylphenyl-, 2-Methoxyphenyl-, 3-Methoxyphenyl-, 4-Methoxyphenyl-, 2-Äthylphenyl-, 3-Äthylphenyl-, 4-Äthylphenyl-, 2-Äthoxyphenyl-, 3-Äthoxyphenyl-, 4-Äthoxyphenyl-, 2,4-Dichlorphenyl-, Naphthyl-gruppe substituierten Homologen.

Die Umsetzung wird bei einer Temperatur von 50 bis 200°C, vorzugsweise 70 bis 180°C, zweckmäßig 80 bis 150°C, drucklos oder unter Druck, diskontinuierlich oder kontinuierlich durchgeführt. Man kann in Abwesenheit organischer Lösungsmittel oder zweckmäßig in Gegenwart von unter den Reaktionsbedingungen inerten, organischen Lösungsmitteln umsetzen. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Halogenkohlenwasserstoffe, insbesondere Chlorkohlenwasserstoffe, z.B. Tetrachloräthylen, 1,1,2,2- oder 1,1,1,2-Tetrachloräthan, Amylchlorid, Dichlorpropan, Methylenchlorid, Dichlorbutan, Isopropylbromid, Chloroform, Äthyljodid, Propyljodid, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, o-, p- und m-Dichlorbenzol, o-, m-, p-Chlortoluol, 1,2,4-Trichlorbenzol. Zweckmäßig verwendet man das Lösungsmittel in einer Menge von 100 bis 10 000 Gewichtsprozent, vorzugsweise von 200 bis 1 200 Gewichtsprozent, bezogen auf Ausgangsstoff II.

Die Reaktion kann wie folgt durchgeführt werden: Ein Gemisch von Ausgangsstoff II und Lösungsmittel wird während 0,1 bis 6 Stunden bei der Reaktionstemperatur gehalten. Dann wird der Endstoff aus dem Reaktionsgemisch in üblicher Weise, z.B. durch fraktionierte Destillation und Kristallisation, abgetrennt.

Die nach dem Verfahren der Erfindung herstellbaren Endstoffe I sind wertvolle Ausgangsstoffe für die Herstellung von Farbstoffen, Pflanzenschutzmitteln und Pharmazeutika. Sie sind wertvolle UV-Lichtschutzmittel. Sie absorbieren in dem für die Bräunung der Haut schädlichen UV-B und C-Bereich (215 - 315 nm) der natürlichen UV-Strahlung. Der Absorptionsbereich der Verbindungen liegt zwischen 215 und 315. Bezüglich der allgemeinen Eigenschaften und Verwendung von Lichtschutzmitteln wird auf Naturwissenschaftliche Rundschau (1972), Band 25, Seiten 89 bis 99, verwiesen.

Die in den folgenden Beispielen angeführten Teile bedeuten Gewichtsteile.

<u>Beispiel 1</u>

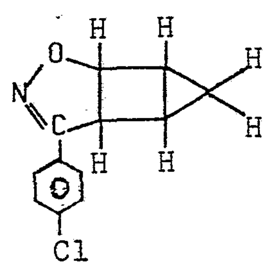

10 Teile 8-[4-Chlorphenyl]-2,3,7-triaza-6-oxa-tricyclo-[5.2.1.$0^{5.9}$]-deca-2,7-dien werden in 100 Teilen Chlorbenzol 15 Stunden bei 130°C am Rückfluß erhitzt. Danach wird das Lösungsmittel im Rotationsverdampfer bei 15 mbar und 40°C abdestilliert. Der Rückstand wird mit Petroläther digeriert und getrocknet. Man erhält 8 Teile 4-[4'-Chlorphenyl]-3-aza-2-oxa-tricyclo-[$3.3.0^{1.5}.0^{6.8}$]-octa-3-en (91 % der Theorie) vom Fp 148°C (aus Ligroin).

Beispiele 2 bis 14

Entsprechend Beispiel 1 werden die in der Tabelle aufgeführten Endstoffe I hergestellt:

Tabelle

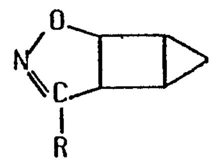

| Beispiel | R | Teile Ausgangsstoff II | Endstoff I Fp in $^oC$ (mbar) | Ausbeute in % der Theorie |
|---|---|---|---|---|
| 2 | $CH_3-$ | 10 | $Kp_{(5)}54$ | 91 |
| 3 | $CH_3-CH_2-$ | 10 | $Kp_{(1)}55$ | 92 |
| 4 | $CH_3-(CH_2)_2-$ | 10 | $Kp_{(1)}66$ | 89 |
| 5 | $CH_3-CH(CH_3)-$ | 10 | $Kp_{(1)}60$ | 94 |
| 6 | $(CH_3)_3C-$ | 10 | $Kp_{(0,5)}53$ | 93 |
| 7 | $CH_3-(CH_2)_3-CH(C_2H_5)-$ | 10 | $Kp_{(1)}110$ | 87 |
| 8 | 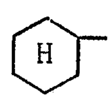 | 10 | $Kp_{(1)}120$ | 90 |
| 9 | $C_6H_5-$ | IO | 51 | 88 |

0046191

Tabelle (Fortsetzung)

| Beispiel | R | Teile Ausgangsstoff II | Endstoff I Fp in °C (mbar) | Ausbeute in % der Theorie |
|---|---|---|---|---|
| 10 | $C_6H_5-CH_2-$ | 10 | Öl, +) | 78 |
| 11 | $p-CH_3-C_6H_4-$ | 10 | 95 | 93 |
| 12 | $p-CH_3O-C_6H_4-$ | 10 | 109 | 89 |
| 13 | 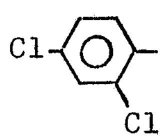 | 10 | 69 | 95 |
| 14 | 1-Naphthyl | 10 | 49 | 92 |

+) $^1$H-NMR ($\delta$ in ppm): 1.0(m,2H); 1.8(m,2H); 3.6(m,3H);4.8(m,1H);7.3(s,5H).

Patentansprüche

1. Isoxazolinverbindungen der Formel

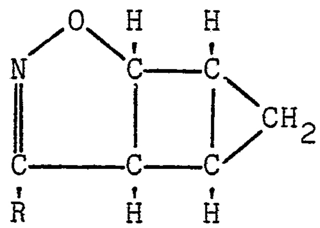

I,

worin R ein Wasserstoffatom, einen aliphatischen, cycloaliphatischen, araliphatischen oder aromatischen Rest bedeutet.

2. Verfahren zur Herstellung von Isoxazolinverbindungen der Formel

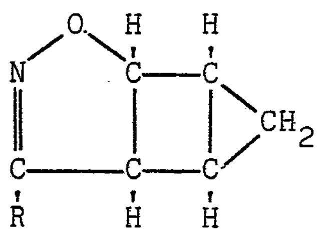

I,

worin R ein Wasserstoffatom, einen aliphatischen, cycloaliphatischen, araliphatischen oder aromatischen Rest bedeutet, dadurch gekennzeichnet, daß man Isoxazolin-azoverbindungen der Formel

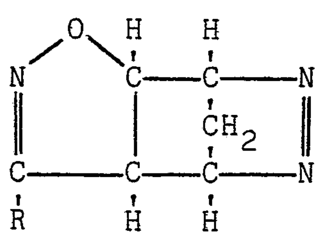

II,

worin R die vorgenannte Bedeutung besitzt, bei einer Temperatur von 50 bis 200°C umsetzt.

Europäisches Patentamt

# EUROPÄISCHER RECHERCHENBERICHT

0046191

Nummer der Anmeldung

EP 81105448.5

| EINSCHLÄGIGE DOKUMENTE | | | KLASSIFIKATION DER ANMELDUNG (Int. Cl.) |
|---|---|---|---|
| Kategorie | Kennzeichnung des Dokuments mit Angabe, soweit erforderlich, der maßgeblichen Teile | betrifft Anspruch | |
| | CHEMICAL ABSTRACTS, Vol. 83, No. 1, 7. September 1975, Columbus, Ohio, USA<br>MUKAI, THOSHIO "1,3-Oxazepine derivatives"<br>Seite 860, Spalte 1, Abstract No. 10186t<br>& Japan. Kokai 74,124,088 (Cl. 16 E54), 27. November 1974, Appl. 73 37,023, 31. März 1973<br>---- | 1 | C 07 D 261/20 |
| | | | RECHERCHIERTE SACHGEBIETE (Int. Cl.) |
| | | | C 07 D 261/00 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| WIEN | 23-10-1981 | HAMMER |